# EUROPEAN PATENT APPLICATION

(11) **EP 4 345 105 A1**
(43) Date of publication of application: **03.04.2024**
(21) Application number: 22815247.6
(22) Date of filing: 31.05.2022
(51) Int. Cl.: C07K 14/605, A61K 38/26, A61P 3/04, A61P 3/10

(54) **POLYPEPTIDE DERIVATIVE HAVING EFFECT OF DUAL TARGETED ACTIVATION OF GLP-1R AND GIPR, PREPARATION METHOD THEREFOR, AND USE THEREOF**

(30) Priority: 01.06.2021 CN 202110608818
(71) Applicant: Nanjing Zhine Medicine Technology Co., Ltd., Nanjing, Jiangsu 210000 (CN)
(72) Inventor: DING, Wei, Nanjing, Jiangsu 210000 (CN); ZHANG, Zhefeng, Nanjing, Jiangsu 210000 (CN)
(74) Representative: karo IP
(86) International application number: PCT/CN2022/096109
(87) International publication number: WO 2022/253202

(57) **Abstract**

The present invention discloses a polypeptide derivative with dual GLP-1R and GIPR targeting agonism, and a preparation method and use thereof. The polypeptide derivative is set forth in the formula (I). The formula (II) is a branched-chain modification structure of a peptide chain. The groups are defined in this specification. The polypeptide derivative and a pharmaceutically acceptable salt thereof that are provided in the present invention can be used in the fields of treatment of diabetes, weight loss, metabolism-related diseases such as NASH, and neurodegenerative disorders.

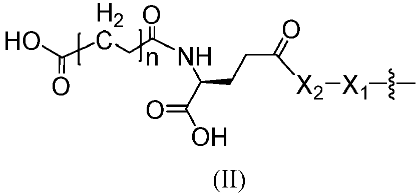

Tyr-Aib-Asp-Gly-Thr-Phe-Thr-Ser-Asp-Tyr-Ser-Ile-Xaa13-Leu-Asp-Lys-Ile-Ala-Gln-Xaa20-
Glu-Phe-Val-Gln-Trp-Leu-Leu-Ala-Gly-Gly-Pro-Ser-Ser-Gly-Ala-Pro-Pro-Pro-Ser-NH2
(I)

## Description

### TECHNICAL FIELD

The present invention relates to the field of medicine technologies, and in particular, to a polypeptide derivative with dual GLP-1R and GIPR targeting agonism, and a preparation method and use thereof.

### BACKGROUND

Glucose-dependent insulinotropic polypeptide (gastric inhibitory polypeptide, GIP) is the first discovered incretin that consists of 42 amino acids and that is secreted by K cells primarily in the proximal small intestine. In early studies, the effect of insulinotropic secretion triggered by acute infusion of GIP in T2DM patients is weakened, limiting the potential of GIP as a target for drug development. However, recent studies have found that the insulinotropic effect of GIP can be restored in T2DM patients after treatment with antidiabetic drugs to improve glycemic control. In addition, GIP can act on bone and adipose tissue to inhibit bone resorption and promote lipid synthesis in adipocytes, which further affects lipid metabolism and fat distribution, with effects independent of the insulinotropic effect. The GIP can also activate GIPR+ neurons in the hypothalamus to reduce food intake and body weight. GIP-treated mice showed improved learning and memory abilities, increased levels of the brain growth factor that protects nerve cell functions, reduced amounts of amyloid associated with Alzheimer's disease, reduced chronic inflammation and oxidative stress, and slower apoptosis of nerve cells.

Glucagon-like peptide-1 (GLP-1) is a peptide hormone secreted by human intestinal L-cells, which can promote insulin secretion and inhibit glucagon secretion, and has the effect of reducing blood sugar concentration. Therefore, GLP-1 is used in the treatment of type II diabetes. GLP-1 binds to its specific receptor GLP-1R to exert physiological effects, and the GLP-1R is widely distributed throughout the body in multiple organs or tissues. Evidence of evidence-based medicine has also confirmed that GLP-1R agonism (GLP-1RA) not only has an effect on the treatment of diabetes and obesity, but also has a protective effect on the cardiovascular system, kidneys, and central nervous system. The widely distributed GLP-1R can regulate the metabolism of key target tissues together with other receptors.

However, naturally occurring GLP-1 and GIP are unstable in vivo and susceptible to rapid degradation by dipeptidyl peptidase-IV (DPP-IV).

Eli Lilly and Company's Tirzepatide (LY3298176), a novel dual GLP-1R and GIPR targeting agonist, is undergoing the clinical phase 3 studies in various indications. On May 14, 2022, the FDA approved Eli Lilly and Company's Mounjaro (tirzepatide) for the improvement of glycemic control of patients with type 2 diabetes, in combination with diet and exercise. Other new polypeptide drugs for dual targets are also under study, such as CN105849122A, CN104470948A, and CN105209485A.

Previous research results have found that a proper combination of dual GLP-1R and GIPR targeting agonist activities is the core of such drugs, and different activity combinations cause different pharmacological effects and adverse reactions. In addition, different branched-chain modifications have different effects on the dual GLP-1R and GIPR targeting agonist activities and the half-life of such drugs. In the art, it is still desired that polypeptides with dual GLP-1R and GIPR targeting agonism have great therapeutic potential in the treatment of diabetes, weight loss, cardiovascular complications of diabetes, NASH, neurodegenerative disorders such as AD and PD, and the like.

### SUMMARY

An objective of the present invention is to discover, through intensive research and creative labor, a polypeptide derivative with dual GLP-1R and GIPR targeting agonism or a salt thereof, and a corresponding pharmaceutical composition. The polypeptide derivative has a long-acting effect in vivo that far exceeds that of comparable polypeptide compounds, and significantly has more potent pharmaceutical activity and better clinical value in certain aspects at an effective dose, so that the polypeptide derivative has great potential in the treatment of diabetes, weight loss, cardiovascular complications of diabetes, metabolic syndrome, NASH, neurodegenerative disorders such as AD and PD, and the like.

The present invention provides a polypeptide derivative with dual GLP-1R and GIPR targeting agonism or a salt thereof. The polypeptide derivative with dual GLP-1R and GIPR targeting agonism is set forth in the formula (I). Xaa₂₀ in a backbone is Lys, and a side chain of Lys (ε-amino group) is linked to X₁ in a branched chain (II) by an amide bond.

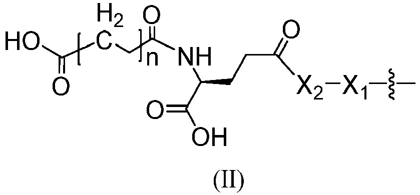

Tyr-Aib-Asp-Gly-Thr-Phe-Thr-Ser-Asp-Tyr-Ser-Ile-Xaa13-Leu-Asp-Lys-Ile-Ala-Gln-Xaa20-
Glu-Phe-Val-Gln-Trp-Leu-Leu-Ala-Gly-Gly-Pro-Ser-Ser-Gly-Ala-Pro-Pro-Pro-Ser-NH2
(I)

In the formula (I), Aib is aminoisobutyric acid, linked to the preceding and following amino acids by amide bonds (peptide bonds); and
Xaa₁₃ is Aib, Tyr, or Ala.

In the formula (II), n is 16 or 18, X₁ and X₂ are both 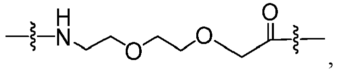 or X₂ is 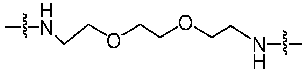 and X₁ is

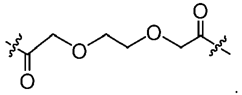

In an implementation of the present invention, in the polypeptide derivative, n in the formula (II) is 16.

In an implementation of the present invention, in the polypeptide derivative, n in the formula (II) is 18.

In an implementation of the present invention, in the polypeptide derivative, in the formula (II), X₁ and X₂ are both

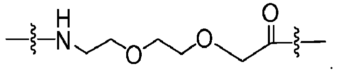

In an implementation of the present invention, in the polypeptide derivative, in the formula (II), X₂ is 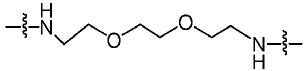 and X₁ is

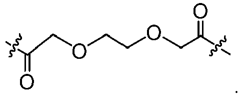

In an implementation of the present invention, the polypeptide derivative is the following formula (1):

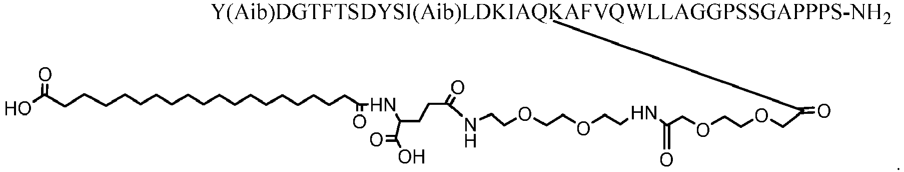

In an implementation of the present invention, the polypeptide derivative is the following formula (2):

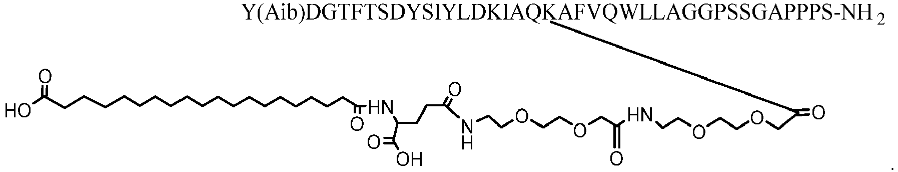

In an implementation of the present invention, the polypeptide derivative is the following formula (3):

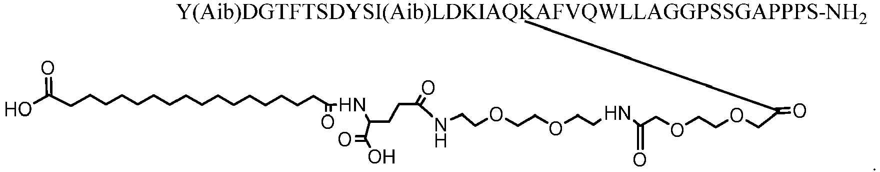

In an implementation of the present invention, the polypeptide derivative is the following formula (4):

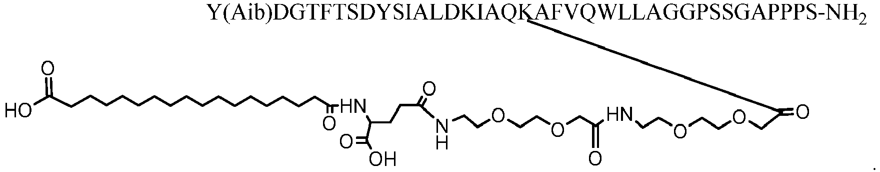

In an implementation of the present invention, the polypeptide derivative is the following formula (5):

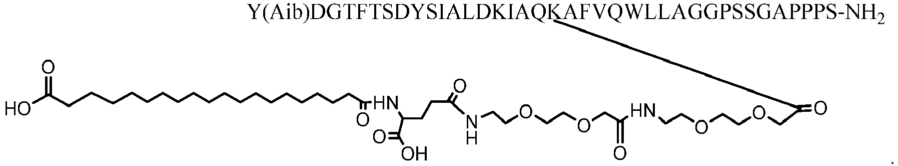

In an implementation of the present invention, the polypeptide derivative is the following formula (6):

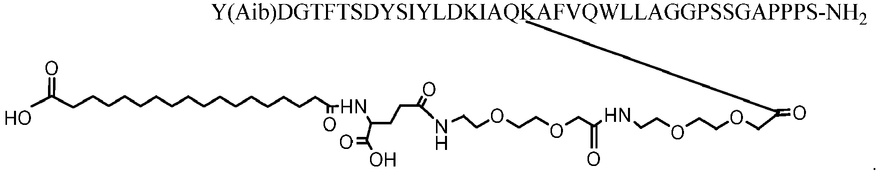

In an implementation of the present invention, the polypeptide derivative is the following formula (7):

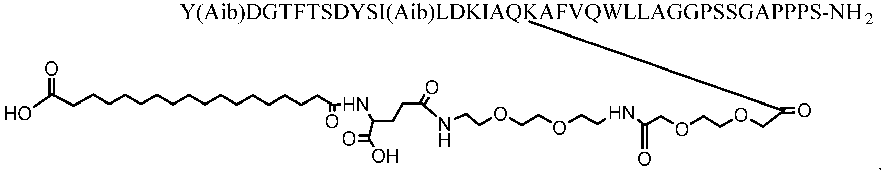

In an implementation of the present invention, the polypeptide derivative is the following formula (8):

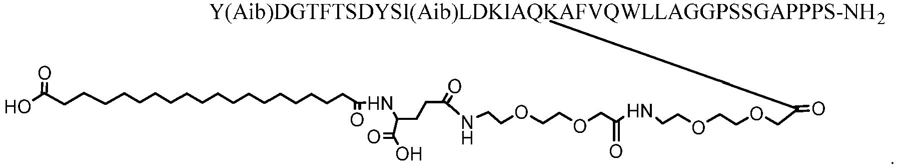

In an implementation of the present invention, the polypeptide derivative is the following formula (9):

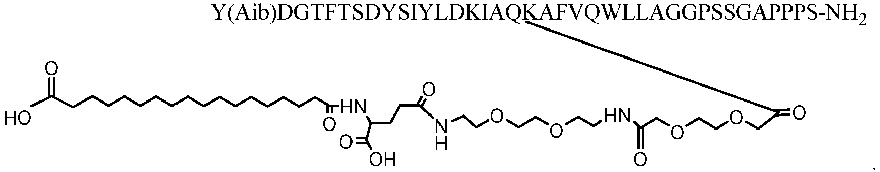

In an implementation of the present invention, the polypeptide derivative is the following formula (10):

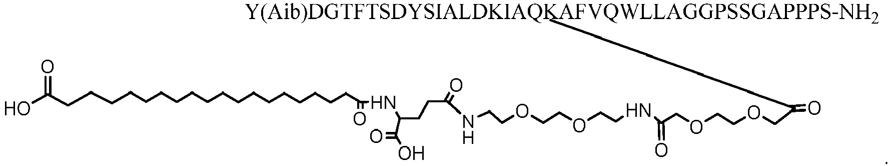

In an implementation of the present invention, glutamine in the branched chain (II) is L-glutamine.

The compound in the present invention may be in a specific form of a geometric isomer or a stereoisomer. The compound in the present invention includes cis- and trans-isomers, (-)- and (+)-enantiomers, (R)- and (S)-enantiomers, diastereomers, (D)-isomers, (L)-isomers, and racemic mixtures and other mixtures thereof, for example, a mixture rich in enantiomers or diastereomers. All such mixtures fall within the scope of the present invention. All these isomers and mixtures thereof are included within the scope of the present invention. In implementations of the present invention, as a preferred implementation of the present invention, amino acids in the polypeptide derivative are preferably L-amino acids.

Except for unnatural amino acids, the natural amino acids in this specification are internationally recognized by single-letter or three-letter abbreviations, such as S or Ser for serine, and P or Pro for proline.

According to another aspect, the present invention provides a preparation method of the polypeptide derivative. The preparation method includes the following step A:
(1) solid-phase or liquid-phase synthesis of branched-chain fragments;
(2) solid-phase synthesis of peptide chain;
(3) coupling of branched-chain fragments and peptide resin; and
(4) cleavage to remove protecting groups and mounted resin, to obtain the polypeptide derivative.

The present invention provides another preparation method of the polypeptide derivative. The preparation method includes the following step B:
(1) solid-phase synthesis of peptide chain;
(2) solid-phase coupling of peptide resin and each branched-chain fragment; and
(3) cleavage to remove protecting groups and mounted resin, to obtain the polypeptide derivative.

In some embodiments, a specific preparation process of step A is described as follows:
(1) Solid-phase or liquid-phase synthesis of branched-chain fragments.

In some embodiments, the solution of the synthesis of branched-chain fragments is as follows:
Fmoc-AEEA-OH ([2-[2-(Fmoc-amino)ethoxy]ethoxy]acetic acid) is condensed and coupled to 2-CTC resin. Fmoc groups are removed with a Pip (piperidine)/DMF (N,N-dimethylformamide) solution or another alkaline solution. The second Fmoc-AEEA-OH is condensed and coupled to the resin. Fmoc groups are removed with a Pip (piperidine)/DMF (N,N-dimethylformamide) solution or another alkaline solution. Fmoc-Glu-OtBu is condensed and coupled to the resin. The tBu-monoprotected octadecanedioic acid is coupled to the resin. The fragments are cut off with 1% TFA (trifluoroacetic acid) in DCM solution to expose carboxyl groups that need to bind to the peptide chain.

In some embodiments, the solution of the synthesis of branched-chain fragments is as follows:
When X₁ is 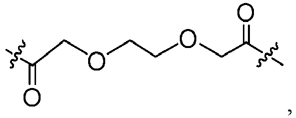 X₂ is 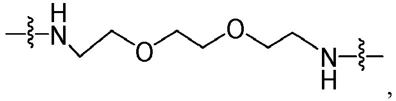 and Xaa₂₀ is a K-mounted branched chain, 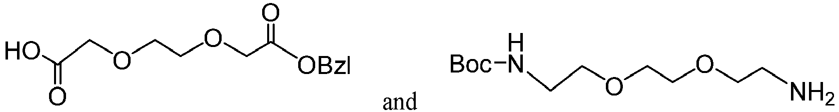 are condensed and coupled in a weak organic alkaline environment, and Boc groups are removed with TFA. Fmoc-Glu-OtBu and the resulting product are condensed and coupled, and Fmoc groups are removed with a Pip solution or another alkaline solution. The tBu-monoprotected octadecanedioic acid is condensed and coupled to the amino group of Glu from the previous step. Finally, benzyl ester is hydrolyzed and removed with palladium-carbon catalyzed hydrogen to expose carboxyl groups that need to bind to the peptide chain.

### (2) Solid-phase synthesis of peptide chain.

In some embodiments, the solution of the synthesis of a backbone is as follows:
The Fmoc-protected amino acids are coupled to the solid-phase synthesized resin in reverse order of the amino acid sequence of the molecular structure in the present invention, and Fmoc groups are removed with a Pip solution or another alkaline solution, until the amino acids on the peptide backbone are all completed. Lys of a to-be-linked side-chain fragment is Alloc-protected (optionally using a raw material Fmoc-Lys(Alloc)-OH) or Dde-protected (optionally using a raw material Fmoc-Lys(Dde)-OH). The terminal serine amide of the molecular structure in the present invention is obtained from resin that can construct amide-type amino acids such as Rink Amide-AM resin, Rink Amide-MBHA resin, Sieber resin, PAM resin, or Rink Amide resin.

### (3) Coupling of branched-chain fragments and peptide resin.

In some embodiments, the solution of the coupling of the branched chain and the peptide chain is as follows:
The peptide resin Lys side chain is deprotected. For example, when a protecting group of the side chain of Xaa₂₀ is Alloc, the side chain is deprotected from Alloc with tetrakis(triphenylphosphine)palladium and PhSiH₃. In another example, when a protecting group of the side chain of Xaa₂₀ is Dde, the side chain is deprotected from Dde with hydrazine hydrate. The branched-chain fragments and the peptide resin are coupled. The first amino acid is deprotected from Fmoc with a Pip solution or another alkaline solution.

### (4) Cleavage to remove protecting groups and mounted resin

Cleave and cut to remove the protecting groups and the mounted resin, to obtain a crude dual-target polypeptide derivative.

In some embodiments, the solution of the cleavage is as follows:
A TFA cleavage cocktail is prepared in a specific ratio. The fully-protected peptide resin is added in the cleavage cocktail to cut off the resin and remove the side chain. Vacuum rotary evaporation is carried out to remove TFA. An ether or alkane solvent is added in the resulting concentrate to precipitate a white solid, which is a target product.

Optionally, the preparation process further includes:
(5) chromatographic purification: carry out multi-step reversed-phase chromatographic purification or ion-exchange chromatographic purification, and finally convert the salt to a salt solution or a free-form solution of a specific acid; and (6) freeze-drying.

In some embodiments, the solution of the synthesis of the backbone in step B is the same as that in step A, but the first amino acid Tyr is Boc-protected at the amino terminal.

In some embodiments, the solution of the solid-phase coupling of the peptide resin and each branched-chain fragment in step B is as follows:
In this solution, the branched-chain fragments are linked to the polypeptide backbone on the resin in reverse order of the structural sequence in the present invention by solid-phase polypeptide synthesis. The side chain of Xaa₂₀ is first deprotected. X₁ is linked to Xaa₂₀, X₂ is linked to X₁, and a glutamate side chain is linked to X₂. Finally, mono-tert-butyl octadecanedioate (or mono-tert-butyl eicosanedioate) is linked to complete the uploading and synthesis of the entire branched chain.

For example, when X₁ and X₂ are both 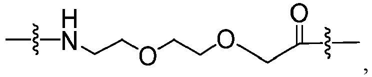 and Xaa₂₀ is a K-mounted branched chain,
Xaa₂₀ (Lys) on the peptide backbone is deprotected from Alloc with tetrakis(triphenylphosphine)palladium and PhSiH₃ or is deprotected from Dde with hydrazine hydrate. Carboxylic acid of Fmoc-AEEA-OH(X₁) is activated by a condensation reagent to be condensed and coupled to the peptide chain, and Fmoc groups are removed with a Pip solution or another alkaline solution. Carboxylic acid of Fmoc-AEEA-OH(X₂) is activated by a condensation reagent to be condensed and coupled to X₁, and the first amino acid is deprotected from Fmoc with a Pip solution or another alkaline solution. Carboxylic acid of Fmoc-Glu-OtBu is activated by a condensation reagent to be condensed and coupled to X₂, and Fmoc groups are removed with a Pip solution or another alkaline solution. The tBu-monoprotected octadecanedioic acid (or eicosandioic acid) is condensed and coupled to the α-amino group of Glu. In this case, the uploading and synthesis of the entire branched chain are completed.

For example, when X₁ is 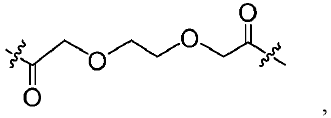 X₂ is 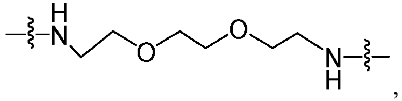 and Xaa₂₀ is a K-mounted branched chain,

X₂ (Lys) on the peptide backbone is deprotected from Alloc with tetrakis(triphenylphosphine)palladium and PhSiH₃ or is deprotected from Dde with hydrazine hydrate. 3,6-dioxaoctanedioic acid (X₁) is preactivated by a condensation reagent, and is then coupled and uploaded to the peptide backbone. Then, Fmoc-monoprotected 3,6-dioxa-1,8-octanediamine (Xz) is condensed and coupled. X₂ is deprotected from Fmoc with a Pip/DMF solution or another alkaline solution. Fmoc-Glu-OtBu is preactivated by a condensation reagent, and is then condensed and coupled. Fmoc groups are removed with a Pip/DMF solution or another alkaline solution. The tBu-monoprotected octadecanedioic acid (or eicosandioic acid) is condensed and coupled to the α-amino group of Glu. In this case, the uploading and synthesis of the entire branched chain are completed.

The condensation and coupling herein are a condensation coupling reaction between carboxylic acid and amino groups. In organic synthesis, a condensation reagent such as DCC/HOBt, DIC/Cl-HOBt, TBTU/DIEA, HBTU/DIEA, PyBOP/DIEA, EDC/HOBt, HATU/DIEA, T3P/DIEA, or DEPBT/TEA may be used according to a specific case (for example, solid-phase polypeptide synthesis).

Some abbreviations in this specification are defined below.
2-CTC resin: 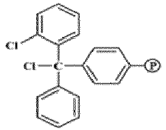
Sieber resin: 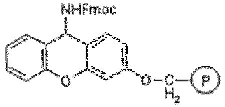
Rink Amide-AM resin: 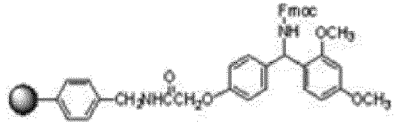
Fmoc: fluorenylmethyloxycarbonyl
EDC: 1-(3-dimethylaminopropyl)-3-ethylcarbodiimide
DIC: N,N'-diisopropylcarbodiimide
HOBt: 1-hydroxybenzotriazole
DCC: N,N'-dicyclopropylcarbodiimide
Cl-HOBt: 6-chloro-1-hydroxybenzotriazole
HBTU: O-benzotriazole-N,N,N',N'-tetramethyluronium hexafluorophosphate
TBTU: O-Benzotriazole-N,N,N',N'-tetramethyluronium tetrafluoroborate
PyBop: benzotriazol-1-yl oxytripyrrolidinylphosphate hexafluorophosphate
HATU: 2-(7-azabenzotriazole)-N,N,N',N'-tetramethyluronium hexafluorophosphate
TIS: Triisopropylsilane
DIEA: diisopropylethylamine
Pip: piperidine (hexahydropyridine)
OtBu: tert-butyl ester
DCM: dichloromethane
MTBE: methyl tertiary butyl ether
Et₂O: ethyl ether
NMP: N-methylpyrrolidone
Bzl: benzyl
TEA: triethylamine
DEPBT: 3-(diethoxyphosphoryloxy)-1,2,3-benzotriazin-4-one
Dde: 1-(4,4-dimethyl-2,6-dioxocyclohexylidene)ethyl
T3P: 1-propylphosphonic anhydride
Alloc: allyloxycarbonyl
tBu: tert-butyl
Pd[P(C₆H₅)₃]₄: tetrakis(triphenylphosphine)palladium
PhSiH₃: phenylsilane

According to a third aspect, the present invention discloses a pharmaceutical composition, including the compound or the pharmaceutically acceptable salt thereof in the present invention as an active ingredient or a main active ingredient, and a pharmaceutically acceptable carrier.

The pharmaceutically acceptable salt forms a part of the present invention. Properly, the "pharmaceutically acceptable salt" includes a free-form compound of the present invention, a conventional non-toxic salt of the compound of the present invention formed through the reaction of inorganic or organic acids, and a conventional non-toxic salt of the compound of the present invention formed through the reaction of inorganic or organic alkalis. For example, the pharmaceutically acceptable salt includes a salt from an inorganic acid such as hydrochloric acid, hydrobromic acid, sulfuric acid, phosphoric acid, or nitric acid, and also includes a salt from an organic acid such as acetic acid, propionic acid, succinic acid, glycolic acid, stearic acid, lactic acid, malic acid, tartaric acid, citric acid, ascorbic acid, maleic acid, hydroxymaleic acid, phenylacetic acid, glutamic acid, benzoic acid, salicylic acid, p-aminobenzenesulphonic acid, fumaric acid, toluenesulphonic acid, methylsulphonic acid, ethylenedisulphonic acid, oxalic acid, hydroxyethanesulphonic acid, or trifluoroacetic acid. In another example, the pharmaceutically acceptable salt includes a salt from an inorganic alkali such as sodium, potassium, calcium, magnesium, zinc, or iron, and also includes a salt from an organic alkali such as ammonia, arginine, lysine, citrulline, or histidine. The salt in the present invention includes an acid- or alkali-containing salt formed by the reaction of small amounts of acid or alkali compounds used for pharmacological purposes. The pharmaceutically acceptable salt is preferably a sodium-containing salt.

An objective of the present invention is to study and develop a clinically usable pharmaceutical formulation based on the polypeptide derivative with dual GLP-1R and GIPR targeting agonism of the present invention. The formulation may further include a buffer, a preservative, an isotonic agent, a co-solvent, a tonicity agent, a chelating agent, a stabilizer, an antioxidant, a surfactant, a pH regulator, and the like, in a concentration of 0.01 mg/mL to 50 mg/mL. The pH of the formulation is 3.0 to 9.0.

In an implementation of the present invention, the pharmaceutical formulation is an aqueous formulation, namely, an aqueous solution, usually a solution, emulsion, or suspension.

In another implementation, the pharmaceutical formulation is a freeze-dried formulation that needs to be fully dissolved in a solvent and/or diluent before use.

In another implementation, the pharmaceutical formulation is a ready-to-use dried formulation that does not require pre-dissolution, for example, spray inhalation freeze-dried powder.

In another implementation of the present invention, the selection of a pH range of the pharmaceutical formulation is critical and affects the solubility and stability of the polypeptide derivative with dual GLP-1R and GIPR targeting agonism, causing physical aggregation or adsorption of the polypeptide under some specific conditions. In an implementation of the present invention, the pH of the pharmaceutical formulation is 3.0 to 5.0. In an implementation of the present invention, the pH of the pharmaceutical formulation is 7.0 to 8.0. In an implementation of the present invention, the pH of the pharmaceutical formulation is 7.5 to 8.5. In another implementation of the present invention, the pH of the pharmaceutical formulation is 5.0 to 7.5.

In a further implementation of the present invention, the buffer is disodium hydrogen phosphate, sodium acetate, or the like; the preservative is phenol, o-cresol, m-cresol, p-cresol, or the like; the isotonic agent is sodium chloride salt, sugar or sugar alcohol, amino acid, propylene glycol, mannitol, or the like; the co-solvent is mannitol, propylene glycol, PEG, glycerin, Tween, ethanol, or the like; the tonicity agent is sodium chloride salt, propylene glycol, glycerin, mannitol, or the like; the chelating agent is EDTA, citrate, or the like; the stabilizer is creatinine, glycine, niacinamide, PEG, or the like; the antioxidant is sodium bisulfite, sodium sulfite, cysteine, methionine, or the like; the surfactant is polysorbate, glycerin, mannitol, or the like; and the pH regulator is hydrochloric acid, phosphoric acid, sulfuric acid, sodium hydroxide, potassium hydroxide, or the like.

Other ingredients may be present in the pharmaceutical formulation including the polypeptide derivative in the present invention as required (for example, long-term stability) by the pharmaceutical formulation, including an emulsifier, metal ions, an oil carrier, protein (such as human serum albumin, gelatin, or proteins), amphoteric ions (such as arginine, glycine, lysine, histidine, betaine, and taurine), and other additives of the pharmaceutical formulation.

The term "pharmaceutically acceptable carrier" refers to any formulation carrier or medium that is capable of delivering an effective amount of the active substance of the present invention, does not interfere with the biological activity of the active substance, and has no toxicological side effects on the host or patient. Typical carriers include water, oil, liposome, and the like.

For drugs or pharmacologically active agents, the term "effective amount" or "therapeutically effective amount" means a sufficient amount of a drug or agent that is non-toxic but achieves a desired effect.

The term "active ingredient", "therapeutic agent", "active substance", or "active agent" means a chemical entity that is effective in treating a target disorder, disease, or condition.

"Optional" or "optionally" means that an event or condition subsequently described may, but is not required to, occur, and that the description includes a case in which the event or condition occurs as well as a case in which the event or condition does not occur.

According to a fourth aspect, the present invention provides use of the polypeptide derivative with dual GLP-1R and GIPR targeting agonism and the pharmaceutically acceptable salt thereof in prevention and/or treatment of diseases. In a preferred technical solution, the diseases include metabolic syndrome, obesity, diabetes, obesity-related diseases, and diabetes-related diseases. The diabetes includes a group of metabolic diseases characterized by hyperglycemia due to defects in insulin secretion, insulin action, or both. The diabetes is categorized into type I diabetes, type II diabetes, and gestational diabetes based on a disease mechanism.

In the present invention, the polypeptide derivative with dual GLP-1R and GIPR targeting agonism and the pharmaceutically acceptable salt thereof can combine effects of GIP (for example, on fat metabolism and weight loss, and on blood sugar) with effects of GLP-1 (for example, on blood sugar levels and food intake). Therefore, the polypeptide derivative and the pharmaceutically acceptable salt thereof can be used to accelerate the elimination of excess adipose tissue, induce sustained weight loss, and improve blood sugar control. The dual GLP-1R and GIPR targeting agonism may also cause the reduction of cardiovascular risk factors, such as high cholesterol, further, high LDL cholesterol.

In the present invention, the polypeptide derivative with dual GLP-1R and GIPR targeting agonism and the pharmaceutically acceptable salt thereof can also be used for treatment of insulin resistance, impaired glucose tolerance, prediabetes, elevated fasting glucose, type II diabetes, hypertension, dyslipidemia (or a combination of these metabolic risk factors), atherosclerosis, arteriosclerosis, coronary artery disease, peripheral arterial disease, and stroke. These are all diseases that may be associated with obesity. However, effects of the compounds used in the present invention on these diseases may be exerted as effects on body weight to implement full or partial mediation, or may be independent of the effects.

In some implementations, the polypeptide derivative with dual GLP-1R and GIPR targeting agonism and the pharmaceutically acceptable salt thereof in the present invention can be used in the treatment of obesity-related inflammation, obesity-related gallbladder diseases, obesity-induced sleep apnea, and other obesity-related diseases.

Preferably, the present invention also includes use in the preparation of drugs for the treatment of type II diabetes with delayed efficacy and/or for the prevention of type II diabetes deterioration, and a method for improving blood sugar control in patients with type II diabetes, including administering an effective amount of the polypeptide derivative to a patient in need thereof as an adjunct to diet and exercise.

In the present invention, the polypeptide derivative with dual GLP-1R and GIPR targeting agonism and the pharmaceutically acceptable salt thereof have positive preventive and therapeutic implications for NASH that is associated with multiple metabolism-related factors and has complex pathogenesis. Insulin resistance and disorders of fat metabolism cause early damage to the liver, resulting in fat accumulation in liver cells (NAFLD). As the disease progresses and the body's immune regulation develops, the liver cells have an inflammatory response, to form fibrosis, ultimately leading to symptoms of end-stage liver disease such as cirrhosis. In the present invention, the polypeptide derivative with dual GLP-1R and GIPR targeting agonism can effectively regulate blood sugar levels involved in metabolism, to fully or partially mediate NASH, or may be independently of these effects.

The metabolic syndrome in the present invention may be diabetes or a diabetes-related disease, or obesity or an obesity-related disease, or NAFLD, NASH, or a related disease. The relationship between obesity and diabetes is well known. Therefore, these diseases may be combined together instead of being independent of each other or mutually exclusive. NAFLD, NASH, or a related disease may not be independent of each other or mutually exclusive because of complex mechanism and involvement in obesity and diabetes.

In the present invention, the polypeptide derivative with dual GLP-1R and GIPR targeting agonism and the pharmaceutically acceptable salt thereof have positive preventive and therapeutic implications for neurodegenerative disorders such as AD (Alzheimer's disease) or PD (Parkinson's disease).

According to a fifth aspect, it has been found through experimental studies of the present invention that the polypeptide derivative with dual GLP-1R and GIPR targeting agonism of the present invention, with conserved or non-conserved amino acid substitutions and branched chain modifications based on the related art, surprisingly has an excellent long-acting effect in vivo, and a half-life significantly and statistically superior to that of the control Tirzepatide. According to disclosed data, an average clinical half-life of the control Tirzepatide in humans is 5.0 days. According to the results of SD rat pharmacokinetic experiments, it can be deduced that a half-life of a polypeptide derivative (1) in humans is 7.88 days, that is, the polypeptide derivative (1) can be administered to patients once every half a month, which helps clinical application.

According to a sixth aspect, it has been found through experimental studies of the present invention that the polypeptide derivative with dual GLP-1R and GIPR targeting agonism of the present invention, with conserved or non-conserved amino acid substitutions and branched chain modifications based on the related art, can effectively activate GIPR and GLP-1R, and provides dual-target agonistic activity with appropriate activity matching and pharmacodynamic effects, which are similar to pharmacodynamic effects of the control Tirzepatide. In addition, it has been unexpectedly found that some active pharmacodynamic effects of the polypeptide derivative are significantly and statistically stronger than those of Tirzepatide, for example, reduction of TC (total cholesterol).

### DETAILED DESCRIPTION

### Example 1 Preparation of polypeptide derivative (1)

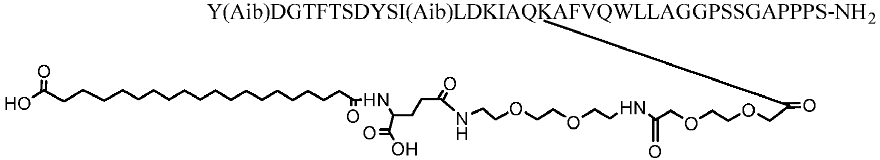

### MALDI-TOF-MS: [M+H] 4800.51

(1) Liquid-phase synthesis of branched-chain fragments. 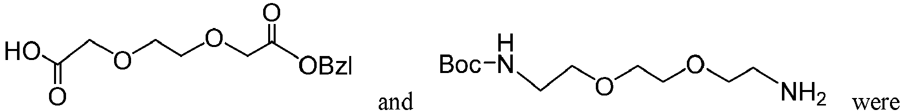 condensed and coupled with isobutyl chlorocarbonate in triethylamine, and Boc groups were removed with TFA. Fmoc-Glu-OtBu and the resulting product were condensed and coupled with TBTU/DIEA, and Fmoc groups were removed with a 20% Pip/NMP solution. The tBu-monoprotected octadecanedioic acid was condensed and coupled to the amino group of Glu from the previous step. Finally, benzyl ester was hydrolyzed and removed with palladium-carbon catalyzed hydrogen to obtain the branched-chain fragments. 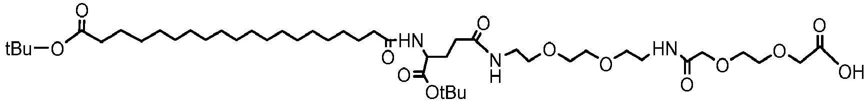
(2) Solid-phase synthesis of peptide chain.

The Fmoc-protected amino acids were coupled to the solid-phase synthesized Rink Amide-MBHA resin from the C-terminus to the N-terminus of the amino acid sequence of the peptide chain of the polypeptide derivative (1), and Fmoc groups were removed with a 20% Pip/NMP solution, until the amino acids on the GLP-1 backbone are all completed. Lys of a to-be-linked side-chain fragment was condensed to the peptide resin from Fmoc-Lys(Dde)-OH.

### (3) Coupling of branched-chain fragments and peptide resin.

Dde groups were removed with a 2% hydrazine hydrate/ethanolamine solution. The branched-chain fragments in step (1) and the peptide resin in step (2) were coupled by a condensation reagent HATU/DIEA. The first amino acid was deprotected from Fmoc with a 20% Pip/DMF solution.

### (4) Cleavage to remove protecting groups and mounted resin.

A cleavage cocktail was prepared in a specific ratio (TFA:TIS:H₂O:EDT = 96:2:1:1). The fully-protected peptide resin was added in the cleavage cocktail to cut a target compound off the resin and remove the side chain. Vacuum rotary evaporation was carried out to remove TFA. Et₂O was added in the resulting concentrate to precipitate a white solid, which is a crude target product.

### (5) Chromatographic purification

Multi-step reversed-phase chromatographic purification or ion-exchange chromatographic purification was carried out, and finally the salt was converted to a sodium-containing salt solution.

### (6) Freeze-drying

Freeze-drying was carried out to obtain the trifluoroacetic acid polypeptide derivative (1), a white or off-white sparse solid.

### Example 2 Preparation of polypeptide derivative (1)

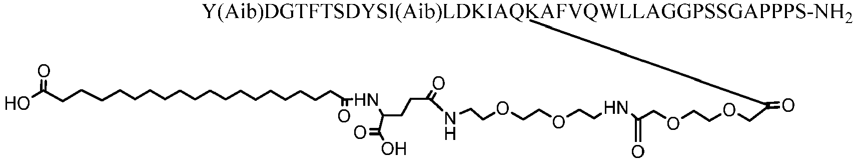

### MALDI-TOF-MS: [M+H] 4800.53

### (1) Solid-phase synthesis of peptide chain.

The Fmoc-protected amino acids were coupled to the Rink Amide-AM resin in reverse order of the amino acid sequence of the peptide chain of the polypeptide derivative (1) with PyBOP/DIEA or DIC/Cl-HOBt, and Fmoc groups were removed with a 20% Pip/DMF solution, until the peptide chain is completed with all the amino acids. Lys of a to-be-linked side-chain fragment was Alloc-protected, that is, Fmoc-Lys(Alloc)-OH. The first amino acid Tyr was from Boc-Tyr(tBu)-OH.

### (2) Solid-phase coupling of peptide resin and each branched-chain fragment.

The peptide resin Lys side chain was deprotected from Alloc with tetrakis(triphenylphosphine)palladium Pd[P(C₆H₅)₃]₄ and a phenylsilane PhSiH₃/DMF solution. 3,6-dioxaoctanedioic acid was preactivated by HATU/DIEA, and was then coupled and uploaded to the peptide resin. Fmoc-monoprotected 3,6-dioxa-1,8-octanediamine was condensed and coupled. Fmoc groups were removed with 20% Pip/DCM. Fmoc-Glu-OtBu was preactivated by a condensation reagent HATU/DIEA, and was then condensed and coupled. Fmoc groups were removed with 20% Pip/DCM. The tBu-monoprotected eicosandioic acid was condensed and coupled to the α-amino group of Glu. In this case, the uploading and synthesis of the entire branched chain were completed.

### (3) Cleavage to remove protecting groups and mounted resin, to obtain the polypeptide derivative.

A cleavage cocktail was prepared in a specific ratio (TFA:TIS:H₂O:phenol = 96:1.5:1.5:1). The fully-protected peptide resin was added in the cleavage cocktail to cut a target compound off the resin and remove the side chain. Vacuum rotary evaporation was carried out to remove TFA. MTBE was added in the resulting concentrate to precipitate a white solid, which is a crude target product.

### (4) Chromatographic purification

Multi-step reversed-phase chromatographic purification was carried out, and finally the salt was converted to an acetic acid salt solution.

### (5) Freeze-drying

Freeze-drying was carried out to obtain the acetic acid polypeptide derivative (1), a white or off-white sparse solid.

Some other compounds described in the present invention were synthesized with reference to the synthesis steps described in Example 1, Example 2, and this specification, respectively:
polypeptide derivative (2) MALDI-TOF-MS: [M+H] 4878.59;
polypeptide derivative (3) MALDI-TOF-MS: [M+H] 4786.52;
polypeptide derivative (4) MALDI-TOF-MS: [M+H] 4772.41;
polypeptide derivative (5) MALDI-TOF-MS: [M+H] 4786.44;
polypeptide derivative (6) MALDI-TOF-MS: [M+H] 4864.55;
polypeptide derivative (7) MALDI-TOF-MS: [M+H] 4786.56;
polypeptide derivative (8) MALDI-TOF-MS: [M+H] 4800.57;
polypeptide derivative (9) MALDI-TOF-MS: [M+H] 4864.50; and
polypeptide derivative (10) MALDI-TOF-MS: [M+H] 4786.43.

### Example 3 SD rat pharmacokinetic experiment

The polypeptide derivative (1) and Tirzepatide were subcutaneously administered to male rats respectively in a single dose, blood samples of the male rats were then collected at different time points, and a concentration of the compound in plasma was determined by LC-MS/MS and related pharmacokinetic parameters were calculated, to investigate the pharmacokinetic characteristics of the compound in the rats.

16 male SD rats were randomly divided into two groups of eight rats each based on body weights. The rats were fasted for 12 h to 14 h, but water was allowed, one day before the administration. The rats were given food 4 h after the administration.

| Group | Quantity | Administration information | | | | | | |
|---|---|---|---|---|---|---|---|---|
| | Male | Substance on trial | Dose for administration (mg/kg) | Concentration for administration (mg/mL) | Volume for administration (mL/kg) | Collected sample | Mode of administration | Solvent |
| G1 | 8 | Drug on trial (1) | 1.35 | 0.54 | 2.5 | Plasma | SC | Saline (colorless clear solution) |
| G2 | 8 | Control Tirzepatide | 1.35 | 0.54 | 2.5 | Plasma | SC | Saline (colorless clear solution) |

Sample collection: Before and after the administration, 0.1 mL of blood was sampled from the orbit of each rat under isoflurane anaesthesia, and then added in an EDTAK2 centrifuge tube in an ice bath to be centrifuged at 4°C at 5000 rpm for 10 min. Then, plasma was collected.

Time points for blood collection of SC groups are 0, 5 min, 15 min, 30 min, 1 h, 2 h, 4 h, 6 h, 8 h, 24 h, 28 h, 32 h, and 48 h. All plasma samples were stored at -80°C before analysis and testing.

The software Analyst 1.5.1 (Applied Biosystem) was used for data collection and system control. Automatic chromatographic peak integration was used for the sample. The sample concentration was regressed with a ratio of the sample peak area to the internal standard peak area as an index. Linear regression was used with a weighting factor of 1/X². Pharmacokinetic parameters were analyzed and processed with a non-compartmental model by using WinNonlin Professional v6.3 (Pharsight, USA). C*ₘₐₓ* is a measured maximum plasma concentration. An area AUC_{*(0*→*t)*} under a plasma concentration-time curve is obtained through ladder-shape calculation. T*ₘₐₓ* is a time of a peak plasma concentration after the administration. The experimental data was expressed as "mean±standard deviation" (Mean±SD, n ≥ 8) or "mean" (Mean, n = 8).

Based on the plasma concentration data, the pharmacokinetic parameters after the administration calculated with the on-compartmental model by using WinNonlin V6.3 are shown in the following table.

### Polypeptide derivative (1) group

| **PK Parameters** | | **Mean** | **SD** | **RSD (%)** |
|---|---|---|---|---|
| Kₑₗ | h⁻¹ | 0.0421 | 0.0055 | 13 |
| T_{1/2} | h | 16.7 | 2.3 | 14 |
| tₘₐₓ | h | 25.3 | 2.3 | 9.1 |
| Cₘₐₓ | ng·mL⁻¹ | 2069 | 200 | 9.7 |
| AUC₀₋ₜ | h·ng·mL⁻¹ | 70390 | 8775 | 12 |
| AUC_{0-inf} | h·ng·mL⁻¹ | 89671 | 11581 | 13 |
| AUMC₀₋ₜ | h·h·ng·mL⁻¹ | 1621331 | 197650 | 12 |
| AUMC_{0-inf} | h·h·ng·mL⁻¹ | 3022264 | 594504 | 20 |
| MRT_{PO} | h | 33.6 | 3.85 | 11 |

### Tirzepatide group

| **PK Parameters** | | **Mean** | **SD** | **RSD (%)** |
|---|---|---|---|---|
| Kₑₗ | h⁻¹ | 0.0680 | 0.016 | 24 |
| T_{1/2} | h | 10.6 | 2.7 | 25 |
| tₘₐₓ | h | 20.0 | 10.6 | 53 |
| Cₘₐₓ | ng·mL⁻¹ | 1535 | 186 | 12 |
| AUC₀₋ₜ | h·ng·mL⁻¹ | 46728 | 5814 | 12 |
| AUC_{0-inf} | h·ng·mL⁻¹ | 52116 | 8169 | 16 |
| AUMC₀₋ₜ | h·h·ng·mL⁻¹ | 1003190 | 179813 | 18 |
| AUMC_{0-inf} | h·h·ng·mL⁻¹ | 1352210 | 368768 | 27 |
| MRT_{PO} | h | 25.6 | 3.19 | 12 |

### Example 4 Glucose-lowering, weight-loss, and fat-loss experiments in ob/ob mice

32 ob/ob mice were uniformly divided into four groups of eight mice each based on fasting body weight, and fasting serum TC and LDL, respectively a model control group, a subject sample group (1.35 mg/kg), a positive control group (Tirzepatide, 1.35 mg/kg), and a normal control group (solvent) of eight conventionally fed mice.

The mice were subjected to subcutaneous injection in the back once every three days for a total of eight times (D0, D3, D6, D9, D12, D15, D18, and D21). Saline was administered to the mice in the model control group and the normal control group in a dose of 0.15 mL/20 g. The drugs were administered to the mice in the subject sample group and the positive control group in a dose of 0.312 mg/kg.
(1) Body weight: The body weights of the mice before grouping were measured once after fasting for 12 h, but water was allowed. Then, the mice were uniformly grouped based on the body weights, and TC and LDL. After grouping, the body weights were measured once a day since D0.
(2) Lee's index: Body lengths of the mice were measured once before the first administration and once on D23. The body length is defined as a maximum straight-line length from the tip of the nose to the anus. The position of a mouse was adjusted to be in a stretched state, and the body length of the mouse was read with a ruler.
   Lee's index = [body weight (g) × 10³/body length (cm)]^{1/3}.
(3) Measurement of glucose (GLU) in blood: Once every three days.
(4) Blood biochemistry tests (TG, TC, LDL, and HDL): Measured once before grouping, once on D11, and once on D23.
(5) Fat-weight coefficient: After euthanasia of the mice, abdominal subcutaneous fat, perigonadal fat, and scapular subcutaneous fat were removed and weighed separately, and the fat-weight coefficient was calculated.
(6) Food intake and water intake: Measured once a day starting from D0 (except for the fasting time period).

The experimental results are shown as follows:

### (1) Body weight:

An initial body weight of the polypeptide derivative (1) group is lower than that of the model control group (D0, p < 0.05) and is not statistically different from that of the Tirzepatide group. Body weights of the mice in the polypeptide derivative (1) group and the Tirzepatide group all decrease on D1 and increase slowly during the experimental period (D1 to D23), and are all significantly lower than those of the model control group (p < 0.01). A mean body weight of the polypeptide derivative (1) group is slightly higher than that of the Tirzepatide group, but there is no statistical difference.

### Record table of body weight of experimental animals

| Date | Normal control group | Model control group | Polypeptide derivative (1) | Tirzepatide |
|---|---|---|---|---|
| 0 | 15.68±1.23** | 18.06±1.17 | 16.01±1.77* | 17.96±2.01 |
| 1 | 16.60±1.38** | 19.01±1.09 | 15.81±1.59** | 17.09±1.61** |
| 2 | 17.39±1.19** | 20.31±1.35 | 15.98±1.49** | 16.91±1.55** |
| 3 | 18.20±1.12** | 20.51±1.42 | 15.93±1.64** | 17.01±2.24** |
| 4 | 17.29±1.55** | 20.95±1.47 | 17.09±1.85** | 17.55±2.03** |
| 5 | 18.85±1.61** | 21.21±1.46 | 16.38±1.73** | 17.49±2.19** |
| 6 | 19.11±1.69** | 22.59±1.52 | 17.28±2.04** | 18.21±2.42** |
| 7 | 19.31±1.59** | 22.60±1.39 | 16.36±2.26** | 16.91±1.75** |
| 8 | 19.54±1.90 | 23.28±1.39 | 16.94±2.00** | 17.49±2.07** |
| 9 | 19.88±1.57** | 24.04±1.58 | 17.69±1.76** | 17.96±2.35** |
| 10 | 19.94±1.62** | 24.56±1.15 | 17.03±1.64** | 17.25±2.17** |
| 11 | 17.65±1.61** | 22.58±1.03 | 16.60±1.66** | 16.85±1.85** |
| 12 | 19.96±1.49** | 24.79±1.02 | 17.66±1.27** | 16.74±2.07** |
| 13 | 20.34±1.52** | 25.49±1.17 | 17.26±1.83** | 17.13±1.75** |
| 14 | 21.41±1.43** | 26.65±1.25 | 18.91±2.02** | 19.01±2.47** |
| 15 | 21.45±1.53** | 26.86±1.61 | 19.75±2.27** | 20.43±2.29** |
| 16 | 21.63±1.39** | 27.88±1.26 | 18.90±2.20** | 19.48±2.51** |
| 17 | 21.16±1.57** | 28.11±1.32 | 19.50±2.27** | 19.84±2.67** |
| 18 | 21.64±1.65** | 28.49±1.27 | 20.39±2.24** | 21.20±2.41** |
| 19 | 22.25±1.44** | 29.55±1.52 | 20.45±2.24** | 20.33±2.73** |
| 20 | 22.35±1.52** | 29.84±1.17 | 20.45±2.24** | 20.33±2.73** |
| 21 | 22.48±1.56** | 29.94±1.62 | 22.10±3.26** | 21.06±2.96** |
| 22 | 22.71±1.70** | 30.43±1.55 | 22.86±3.46** | 21.83±2.96** |
| 23 | 22.76±1.76** | 30.48±1.56 | 22.08±3.14** | 21.35±3.11** |

Compared with the model control group, *p < 0.05, and **p < 0.01. Compared with the corresponding positive drug, #p < 0.05, and ##p < 0.01.

### (2) Lee's index

The Lee's index is a valid indicator of the degree of obesity in rats. The Lee's indexes of all mice increase, with the least increase in the normal control group. Compared with the model control group, the Lee's indexes of the mice in the administration groups slightly decrease. It can be learned from the results that the subject drug and the positive control drug can inhibit the obesity of the ob/ob mice, and the control ability of the subject drug is close to that of the positive control drug with no statistical difference.

**Table of Lee's index of animals**

| | D0 | D23 |
|---|---|---|
| Normal control group | 12.80±0.26** | 13.72±0.29** |
| Model control group | 13.51±0.32 | 15.58±0.43 |
| Polypeptide derivative (1) | 12.97±0.40* | 14.66±0.50** |
| Tirzepatide | 13.18±0.35 | 14.51±0.48** |

### (3) Random blood glucose:

The mice were subjected to initial blood glucose determination on the third day after the administration. It can be learned from the results that blood glucose concentrations of the mice in the administration groups are significantly lower than those in the normal control group, and are well controlled during D0 to D10. During the experiment, the gradual increased blood glucose concentrations of the mice may be related to their ages and weight gains. Compared with the model control group, the blood glucose values of the mice in the normal control group on D14 to D23 are significantly lower than those of the mice in the model control group, indicating that the ob/ob mice have higher basal blood glucose values. Compared with the mice in the model control group, the subject drug and the positive control drug on D3 to D23 have different degrees of significant hypoglycemic effects. There is no significant difference between the subject drug and the positive control drug (the polypeptide derivative (1)/Tirzepatide).

**Table of blood glucose changes in experimental animals**

| | D3 | D7 | D10 | D14 | D17 | D23 |
|---|---|---|---|---|---|---|
| Normal control group | 8.95±1.62 | 6.79±0.92 | 10.89±2.53 | 10.03±1.26** | 10.28±1.34** | 10.10±1.89 |
| Model control group | 9.06±2.37 | 6.00±0.75 | 9.00±1.70 | 15.51±3.43 | 14.75±3.49 | 11.09±2.49 |
| Polypeptide derivative (1) | 5.03±1.63** | 5.06±2.26 | 6.46±1.30** | 6.96±0.82** | 8.49±0.90** | 7.69±2.49* |
| Tirzepatide | 6.30±1.95* | 4.44±0.93** | 6.16±1.78** | 6.71±1.08** | 8.19±1.05** | 8.46±1.92* |

Compared with the model control group, *p < 0.05, and **p < 0.01. Compared with the corresponding positive drug, #p < 0.05, and ##p < 0.01.

### (4) Blood biochemistry tests

1. TC: Before the test (D0), initial values of the polypeptide derivative (1) group and the model control group are higher than those of other groups of mice of the same strain. By comparing values at different times after the administration, the TC values of the obese mice in the polypeptide derivative (1) group and the Tirzepatide group are significantly reduced, indicating that the subject drug and the positive control drug can significantly reduce the TC content. It can be learned from the results of D11 and D23 that the ability of the polypeptide derivative (1) to reduce TC is similar to that of Tirzepatide. In addition, the polypeptide derivative (1) group with the high initial value has strong ability of reducing TC and has a value close to that of the Tirzepatide group on D23, and the statistical significance is improved significantly from ##p < 0.01 to #p < 0.05.
2. TG: Before the test (D0), initial values of the polypeptide derivative (1) group and the Tirzepatide group are lower than those of other groups of mice of the same strain. By comparing values at different times after the administration, the TG values of the obese mice in the polypeptide derivative (1) group and the Tirzepatide group are significantly reduced, indicating that the subject drug and the positive control drug can significantly reduce the TG content. On D23, the polypeptide derivative (1) is slightly weaker than Tirzepatide in the ability of reducing TG (the polypeptide derivative (1) vs Tirzepatide, p < 0.01).
3. LDL: Before the test (D0), initial values of the polypeptide derivative (1) group and the Tirzepatide group are higher than those of other groups of mice of the same strain. By comparing values at different times after the administration, on D11 and D23 after the administration, the LDL values of the obese mice in the polypeptide derivative (1) group and the Tirzepatide group are significantly reduced and significantly lower than those in the model control group (p < 0.01), and there is no statistical difference between the polypeptide derivative (1) group and the Tirzepatide group.
4. HDL: During the experiment, the HDL levels of the mice in the model control group are higher than those of the mice in the normal control group. Before the administration (D0), the HDL levels of the mice of the same strain are close. Compared with the model control group, on D11 and D23 after the administration, the subject drug and the positive control drug can significantly reduce the HDL level.

It can be learned from the foregoing results that the subject drug and the positive control drug can reduce the TG concentration, TC concentration, LDL concentration, and HDL concentration of animals.

**Table of blood TC content changes in animals**

| | D0 | D11 | D23 |
|---|---|---|---|
| Normal control group | 2.76±0.26* | 3.05±0.79 | 1.98±0.34** |
| Model control group | 3.15±0.41 | 3.54±0.42 | 3.88±0.27 |
| Polypeptide derivative (1) | 3.42±0.44## | 2.71±0.70*# | 2.50±0.34**# |
| Tirzepatide | 2.48±0.19** | 2.02±0.34** | 2.13±0.18** |

Compared with the model control group, *p < 0.05, and **p < 0.01. Compared with the corresponding positive drug, #p < 0.05, and ##p < 0.01.

**Table of blood TG contents in animals**

| | D0 | D11 | D23 |
|---|---|---|---|
| Normal control group | 2.08±0.39* | 2.87±0.73 | 1.66±0.25** |
| Model control group | 2.52±0.24 | 3.28±0.31 | 2.28±0.53 |
| Polypeptide derivative (1) | 1.86±0.27** | 2.27±1.07* | 1.12±0.15**## |
| Tirzepatide | 1.79±0.24** | 1.79±0.45** | 0.78±0.16** |

Compared with the model control group, *p < 0.05, and **p < 0.01. Compared with the corresponding positive drug, #p < 0.05, and ##p < 0.01.

**Table of blood LDL contents in animals**

| | D0 | D11 | D23 |
|---|---|---|---|
| Normal control group | 1.82±0.42* | 2.13±0.98** | 2.32±0.58* |
| Model control group | 1.12±0.63 | 4.69±1.51 | 3.05±0.72 |
| Polypeptide derivative (1) | 2.90±0.79** | 2.05±0.68** | 1.24±0.68** |
| Tirzepatide | 2.49±1.21* | 2.20±0.68** | 1.55±0.56** |

Compared with the model control group, *p < 0.05, and **p < 0.01. Compared with the corresponding positive drug, #p < 0.05, and ##p < 0.01.

**Table of blood HDL contents in animals**

| | D0 | D11 | D23 |
|---|---|---|---|
| Normal control group | 0.40±0.14** | 1.06±0.42** | 1.86±0.54* |
| Model control group | 0.97±0.43 | 2.19±0.47 | 2.64±0.68 |
| Polypeptide derivative (1) | 1.24±0.35# | 1.11±0.27** | 1.28±0.40** |
| Tirzepatide | 0.85±0.35 | 1.30±0.36** | 1.48±0.40** |

Compared with the model control group, *p < 0.05, and **p < 0.01. Compared with the corresponding positive drug, #p < 0.05, and ##p < 0.01.

### (5) Fat-weight coefficient

On day 23 after the administration, the mice were dissected, fat at different parts was separated, and the fat-weight coefficient was calculated according to the formula (fat/weight × 100). It can be learned from the results that the fat-weight coefficients at different parts of the mice in the model control group are significantly higher than those of the mice in the normal control group. The polypeptide derivative (1) can significantly reduce abdominal subcutaneous fat and perigonadal fat of the ob/ob mice, and has no significant difference in effects from the positive control drug Tirzepatide. In addition, the drugs have no effect on the scapular subcutaneous fat-weight coefficient.

**Table of fat-weight coefficient**

| | Abdominal subcutaneous fat | Perigonadal fat | Scapular subcutaneous fat |
|---|---|---|---|
| Normal control group | 0.17±0.04** | 0.86±0.26** | 0.49±0.22** |
| Model control group | 2.13±0.85 | 5.58±0.40 | 1.38±0.25 |
| Polypeptide derivative (1) | 0.84±0.34* | 3.33±0.55** | 1.21±0.13 |
| Tirzepatide | 0.75±0.24** | 3.60±0.68** | 1.16±0.34 |

Compared with the model control group, *p < 0.05, and **p < 0.01. Compared with the corresponding positive drug, #p < 0.05, and ##p < 0.01.

### (6) Food intake and water intake

After the administration, the food intake of the mice decreases significantly. An overall food-intake trend of the mice has a specific correlation with administration frequency. To be specific, the mice have the lowest food intake on the day of administration, and have slowly increased food intake with time, but the overall increase is lower than that of the model control group and the normal control group. The overall increase in food intake of the mice in the later stages of the experiment may be related to their ages and weight gains. It can be learned from the experimental results that the subject drug and the positive control drug can significantly control food intake.

The water intake of the mice in the groups varies greatly. The water intake of the administration groups is lower than that of the normal control group and that of the model control group. It can be clearly observed that the water intake of the mice decreases after the administration, which is the most significant after the first administration. The significant increase in the water intake of the mice in each group on D11 may be related to the subsequent fasting operation, but the water intake of the mice in all the groups increases. It can be learned from the experimental results that the subject drug and the positive control drug can significantly control water intake.

## Claims

1. A polypeptide derivative as set forth in (I), or a geometric isomer, a stereoisomer, or a pharmaceutically acceptable salt thereof, wherein Xaa₂₀ in a backbone is Lys, and a side chain of Lys (ε-amino group) is linked to X₁ in a branched chain (II) by an amide bond, 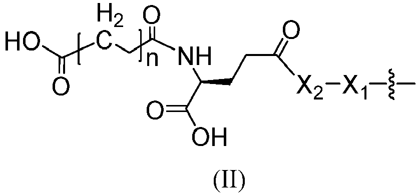 
Tyr-Aib-Asp-Gly-Thr-Phe-Thr-Ser-Asp-Tyr-Ser-Ile-Xaa13-Leu-Asp-Lys-Ile-Ala-Gln-Xaa20-
Glu-Phe-Val-Gln-Trp-Leu-Leu-Ala-Gly-Gly-Pro-Ser-Ser-Gly-Ala-Pro-Pro-Pro-Ser-NH2
(I)
wherein Aib is aminoisobutyric acid, linked to the preceding and following amino acids by amide bonds (peptide bonds);
Xaa₁₃ is Aib, Tyr, or Ala; and
in the formula (II), n is 16 or 18, X₁ and X₂ are both 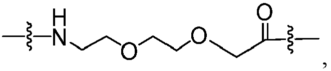 or X₂ is 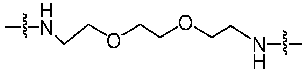 and X₁ is

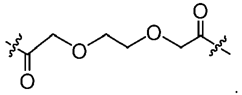

2. The polypeptide derivative, or the geometric isomer, the stereoisomer, or the pharmaceutically acceptable salt thereof according to claim 1, wherein only Xaa₂₀ in the backbone is Lys, and the side chain of Lys (ε-amino group) is linked to a branched chain modified by long-chain fatty acid, wherein X₂ is 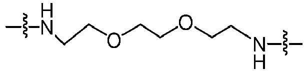 and X₁ is

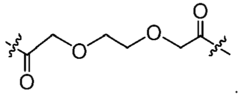

3. The polypeptide derivative, or the geometric isomer, the stereoisomer, or the pharmaceutically acceptable salt thereof according to claim 1, wherein the polypeptide derivative is: 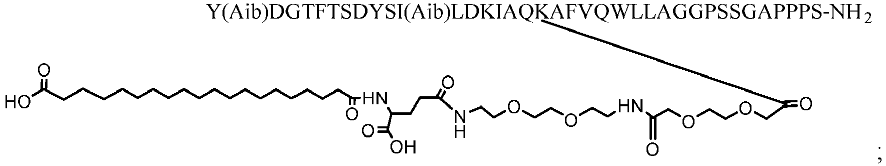 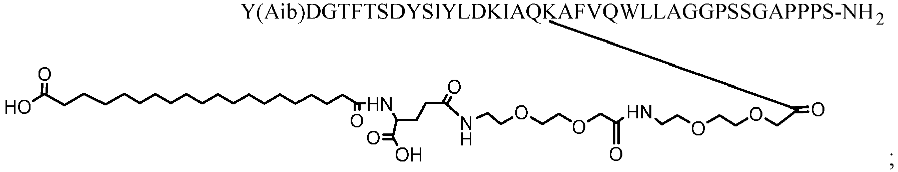 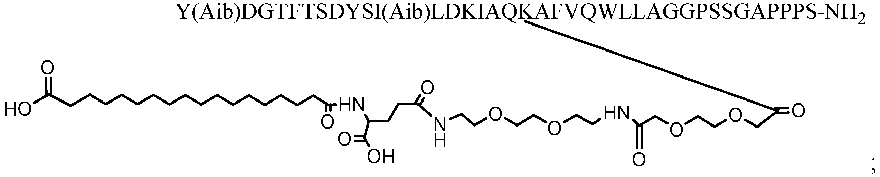 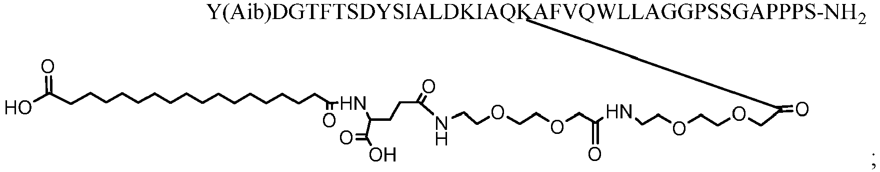 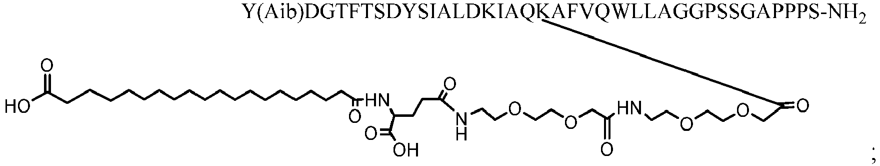 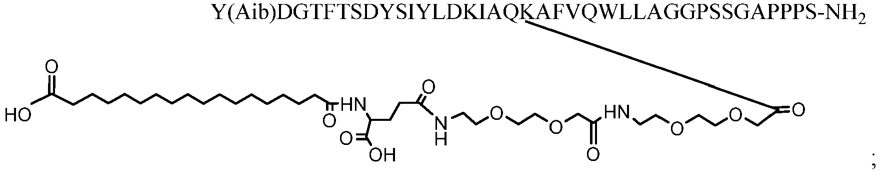 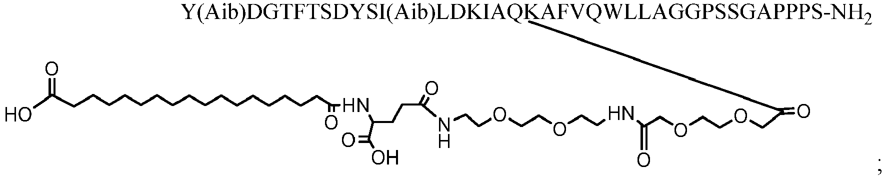 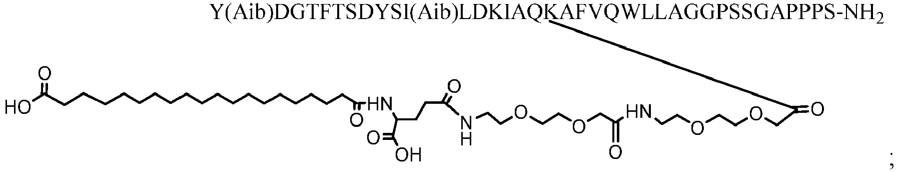 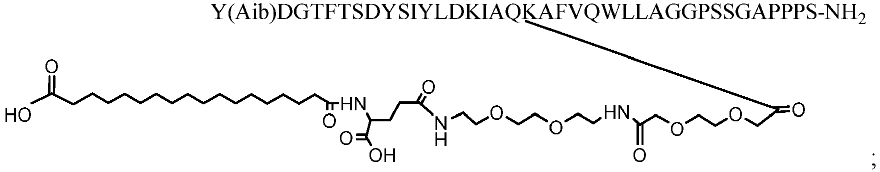 or

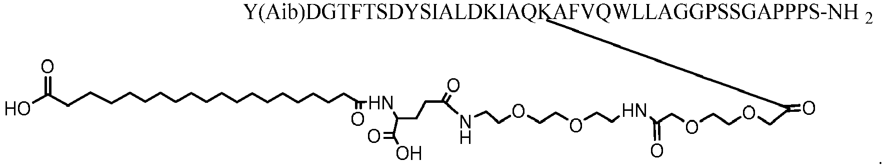

4. The polypeptide derivative, or the geometric isomer, the stereoisomer, or the pharmaceutically acceptable salt thereof according to any one of claims 1 to 3, wherein the amino acids are L-amino acids, and the pharmaceutically acceptable salt is preferably a sodium salt.

5. A preparation method of the polypeptide derivative, or the geometric isomer, the stereoisomer, or the pharmaceutically acceptable salt thereof according to any one of claims 1 to 4, comprising steps of branched-chain fragment synthesis, fully protected polypeptide backbone synthesis, coupling of branched chain and peptide resin, cleavage and deprotection of peptide chain and side chain, chromatographic purification, and freeze-drying; or comprising steps of solid-phase synthesis of peptide chain, solid-phase coupling of peptide resin and each branched-chain fragment, cleavage to remove protecting groups and mounted resin, chromatographic purification, and freeze-drying.

6. A pharmaceutical composition comprising the polypeptide derivative, or the geometric isomer, the stereoisomer, or the pharmaceutically acceptable salt thereof according to any one of claims 1 to 4, wherein the pharmaceutical composition further comprises a pharmaceutically acceptable carrier.

7. Use of the polypeptide derivative, or the geometric isomer, the stereoisomer, or the pharmaceutically acceptable salt thereof according to any one of claims 1 to 4 or the pharmaceutical composition according to claim 6 in drugs for treatment and/or prevention of at least one of the following diseases: metabolic syndrome, obesity, diabetes, obesity-related diseases, and diabetes-related diseases.

8. Use of the polypeptide derivative, or the geometric isomer, the stereoisomer, or the pharmaceutically acceptable salt thereof according to any one of claims 1 to 4 or the pharmaceutical composition according to claim 6 in drugs for treatment and/or prevention of at least one of the following diseases: insulin resistance, impaired glucose tolerance, prediabetes, elevated fasting glucose, type II diabetes, hypertension, dyslipidemia (or a combination of these metabolic risk factors), atherosclerosis, arteriosclerosis, coronary artery disease, peripheral arterial disease, stroke, and other diseases that may be associated with obesity.

9. Use of the polypeptide derivative, or the geometric isomer, the stereoisomer, or the pharmaceutically acceptable salt thereof according to any one of claims 1 to 4 or the pharmaceutical composition according to claim 6 in drugs for treatment and/or prevention of at least one of the following diseases: obesity-related inflammation, obesity-related gallbladder diseases, obesity-induced sleep apnea, and other obesity-related diseases.

10. Use of the polypeptide derivative, or the geometric isomer, the stereoisomer, or the pharmaceutically acceptable salt thereof according to any one of claims 1 to 4 or the pharmaceutical composition according to claim 6 in preparation of drugs for reducing food intake, reducing β cell apoptosis, increasing pancreatic β cell functions, increasing β cell mass, and/or restoring glucose sensitivity to β cells.

11. Use of the polypeptide derivative, or the geometric isomer, the stereoisomer, or the pharmaceutically acceptable salt thereof according to any one of claims 1 to 4 or the pharmaceutical composition according to claim 6 in drugs for treatment and/or prevention of at least one of the following diseases: non-alcoholic fatty liver (NAFL), non-alcoholic steatohepatitis (NASH) and its associated cirrhosis, and hepatocellular carcinoma.

12. Use of the polypeptide derivative, or the geometric isomer, the stereoisomer, or the pharmaceutically acceptable salt thereof according to any one of claims 1 to 4 or the pharmaceutical composition according to claim 6 in drugs for treatment and/or prevention of at least one of the following diseases: neurodegenerative disorders comprising Alzheimer's disease (AD), Parkinson's disease (PD), and the like.
